# EUROPÄISCHE PATENTANMELDUNG

(11) **EP 4 684 737 A1**
(43) Veröffentlichungstag der Anmeldung: **28.01.2026**
(21) Anmeldenummer: 25191166.5
(22) Anmeldetag: 23.07.2025
(51) Int. Cl.: A61B 8/08

(54) **VERFAHREN UND SYSTEM ZUM ERMITTELN EINER POSITION EINER KANÜLENSPITZE**

(30) Priorität: 24.07.2024 DE 102024120960
(71) Anmelder: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(57) **Zusammenfassung**

Die Erfindung betrifft ein Verfahren zum Ermitteln einer Position einer Kanülenspitze im Inneren eines Körpers, aufweisend die Schritte: Bereitstellen eines Ultraschallkopfs eines bildgebenden Ultraschallsystems; Bereitstellen einer Sendeeinrichtung, die mehrere Ultraschallpositionssender und wenigstens einen Befestigungsabschnitt aufweist; Befestigen der Sendeeinrichtung an dem Ultraschallkopf, wobei die Sendeeinrichtung mittels des Befestigungsabschnitts lösbar und in einer definierten Relativposition zu dem Ultraschallkopf an dem Ultraschallkopf befestigt wird; Einkoppeln mehrerer Ultraschallpositionssignale in den Körper, wobei die Ultraschallpositionssignale mittels der mehreren Ultraschallpositionssender eingekoppelt werden; Empfangen der Ultraschallpositionssignale, wobei die Ultraschallpositionssignale mittels eines Sensors empfangen werden, der an einer in dem Körper befindlichen Kanüle angebracht und in einem bekannten axialen Abstand zu deren Kanülenspitze angeordnet ist; Ermitteln von Signallaufzeiten der Ultraschallpositionssignale, wobei die Signallaufzeiten mittels einer Auswerteeinrichtung ermittelt werden, die mit dem Sensor und der Sendeeinrichtung verbunden ist; Ermitteln der Position der Kanülenspitze in Relation zu dem Ultraschallkopf, wobei die Position in Abhängigkeit der Signallaufzeiten, der Relativposition der Sendeeinrichtung zu dem Ultraschallkopf und dem axialen Abstand des Sensors von der Kanülenspitze mittels der Auswerteeinrichtung ermittelt wird. Die Erfindung betrifft zudem ein medizinisches System zum Ermitteln einer Position einer Kanülenspitze.

## Beschreibung

Die Erfindung betrifft ein Verfahren und ein System zum Ermitteln einer Position einer Kanülenspitze im Inneren eines Körpers.

Solche Verfahren und Systeme finden unter anderem im Bereich der Regionalanästhesie Verwendung. Aus dem Stand der Technik ist ein System unter der Bezeichnung "OnVision" bekannt und dazu eingerichtet, die Position der Kanülenspitze in einer Bildebene eines Ultraschallbilds anzuzeigen. Das bekannte System ist vollständig in ein zugrunde liegendes bildgebendes Ultraschallsystem integriert.

Aufgabe der Erfindung ist es, ein Verfahren und ein System der eingangs genannten Art bereitzustellen, die Vorteile gegenüber dem Stand der Technik bieten.

Diese Aufgabe wird durch das Bereitstellen eines Verfahrens mit den Merkmalen des Anspruchs 1 und eines medizinischen Systems mit den Merkmalen des Anspruchs 8 gelöst. Vorteilhafte Weiterbildungen sind in den Unteransprüchen angegeben. Der Wortlaut der Ansprüche wird durch Bezugnahme zum Gegenstand der Beschreibung gemacht.

Das erfindungsgemäße Verfahren weist die Schritte auf: Bereitstellen eines Ultraschallkopfs eines bildgebenden Ultraschallsystems; Bereitstellen einer Sendeeinrichtung, die mehrere Ultraschallpositionssender und wenigstens einen Befestigungsabschnitt aufweist; Befestigen der Sendeeinrichtung an dem Ultraschallkopf, wobei die Sendeeinrichtung mittels des Befestigungsabschnitts lösbar und in einer definierten Relativposition zu dem Ultraschallkopf an dem Ultraschallkopf befestigt wird; Einkoppeln mehrerer Ultraschallpositionssignale in den Körper, wobei die Ultraschallpositionssignale mittels der mehreren Ultraschallpositionssender eingekoppelt werden; Empfangen der Ultraschallpositionssignale, wobei die Ultraschallpositionssignale mittels eines Sensors empfangen werden, der an einer in dem Körper befindlichen Kanüle angebracht und in einem bekannten axialen Abstand zu deren Kanülenspitze angeordnet ist; Ermitteln von Signallaufzeiten der Ultraschallpositionssignale, wobei die Signallaufzeiten mittels einer Auswerteeinrichtung ermittelt werden, die mit dem Sensor und der Sendeeinrichtung verbunden ist; Ermitteln der Position der Kanülenspitze in Relation zu dem Ultraschallkopf, wobei die Position in Abhängigkeit der Signallaufzeiten, der Relativposition der Sendeeinrichtung zu dem Ultraschallkopf und dem axialen Abstand des Sensors von der Kanülenspitze mittels der Auswerteeinrichtung ermittelt wird. Das erfindungsgemäße Verfahren erfordert keine Integration in das bildgebende Ultraschallsystem und ist deshalb besonders universell verwendbar. Anstelle einer software- und/oder hardwareseitigen Integration in das bildgebende Ultraschallsystem sieht das erfindungsgemäße Verfahren die lösbare Befestigung der Sendeeinrichtung an dem Ultraschallkopf des bildgebenden Ultraschallsystems vor. Zur Befestigung an dem Ultraschallkopf weist die Sendeeinrichtung den wenigstens einen Befestigungsabschnitt auf, der lösbar kraft- und/oder formschlüssig an dem Ultraschallkopf befestigbar ist. Die Befestigung erfolgt in einer definierten Relativposition zu dem Ultraschallkopf. Die Ultraschallpositionssender der Sendeeinrichtung sind zum Einkoppeln der mehreren Ultraschallpositionssignale in den Körper eingerichtet. Diese Ultraschallpositionssignale werden mittels des an der Kanüle angebrachten Sensors empfangen, wobei bei einer Ausgestaltung der Erfindung mehrere Sensoren an der Kanüle angebracht sind. Das eigentliche Ermitteln der Position der Kanülenspitze erfolgt mittels der Auswerteeinrichtung. Diese ist mit dem wenigstens einen Sensor und der Sendeeinrichtung verbunden, beispielsweise über jeweils eine drahtgebundene oder drahtlose Verbindung. Zum Ermitteln der Position der Kanülenspitze werden zunächst die Signallaufzeiten der Ultraschallpositionssignale zwischen den einzelnen Ultraschallpositionssendern und dem wenigstens einen Sensor ermittelt. Dabei sind der Fachperson unterschiedliche Herangehensweisen zur Ermittlung von Signallaufzeiten bekannt, so dass auf weitere Details hierzu nicht näher eingegangen werden braucht. Sind die Signallaufzeiten bekannt, kann, insbesondere in Abhängigkeit einer Frequenz und/oder Ausbreitungsgeschwindigkeit der Ultraschallpositionssignale, auf einen jeweiligen Abstand zwischen den einzelnen Ultraschallpositionssendern und dem wenigstens einen Sensor an der Kanülenspitze geschlossen werden. Das Ermitteln der Position der Kanülenspitze erfolgt dann in Abhängigkeit der ermittelten Signallaufzeiten und/oder Abstände, der Relativposition der Sendeeinrichtung zu dem Ultraschallkopf und dem axialen Abstand zwischen dem wenigstens einen Sensor und der Kanülenspitze. Vorzugsweise ist der wenigstens eine Sensor an der Kanülenspitze angeordnet, so dass der axiale Abstand praktisch null oder im Hinblick auf die Genauigkeit der Positionsermittlung vernachlässigbar klein ist. Die Positionsermittlung erfolgt auf Grundlage bekannter geometrischer, im Speziellen trigonometrischer, Beziehungen. Bei Ausgestaltungen mit mehreren Sensoren, sind diese in bekannten axialen Abständen von der Kanülenspitze angeordnet. In diesem Fall kann auch eine Orientierung der Kanüle ermittelt werden. Die Orientierung kann auf der Grundlage bekannter geometrischer, im Speziellen trigonometrischer, Beziehungen mittels des Auswerteeinrichtung ermittelt werden. Zudem erlauben die mehreren Sensoren eine genauere Positionsermittlung. Weiter wird durch die mehreren und unter Umständen auch weiter von der Kanülenspitze entfernten Sensoren vermieden, dass die Kanülenspitze durch das Vorhandensein eines möglichst nah an der Kanülenspitze platzierten Sensors in ihrer Funktion beeinträchtigt wird.

Das erfindungsgemäße Verfahren eignet sich in besonderer Weise zur Verwendung bei einer Regionalanästhesie.

In Ausgestaltung der Erfindung wird wenigstens ein erstes Ultraschallpositionssignal mit einem ersten Ultraschallpositionssender eingekoppelt und ein zweites Ultraschallpositionssignal wird mit einem zweiten Ultraschallpositionssender eingekoppelt. Bei dieser Ausgestaltung weist die Sendeeinrichtung folglich wenigstens zwei Ultraschallpositionssender auf, nämlich den ersten Ultraschallpositionssender und den zweiten Ultraschallpositionssender. Hierdurch erlaubt diese Ausgestaltung des Verfahrens die Ermittlung der Position in Bezug auf zwei Koordinatenachsen, d. h. in einer Ebene. Eine solche ebene Positionsermittlung kann für einige Anwendungsfälle bereits ausreichend sein.

In weiterer Ausgestaltung der Erfindung wird ein drittes Ultraschallpositionssignal mit einem dritten Ultraschallpositionssender eingekoppelt. Bei dieser Ausgestaltung weist die Sendeeinrichtung folglich wenigstens drei Ultraschallpositionssender auf, nämlich den ersten Ultraschallpositionssender, den zweiten Ultraschallpositionssender und den dritten Ultraschallpositionssender. Diese Ausgestaltung der Erfindung erlaubt eine Positionsermittlung der Kanülenspitze in Bezug auf drei Koordinatenachsen. Eine solche räumliche Positionsermittlung hat eine weitergehende Aussagekraft als eine ebene Positionsermittlung. Bei einer weiteren Ausgestaltung weist die Sendeeinrichtung mehr als drei Ultraschallpositionssender auf, beispielsweise vier, fünf, sechs oder mehr als sechs. Dabei wird eine entsprechende Anzahl an Ultraschallpositionssignalen eingekoppelt. Hierdurch kann eine weiter verbesserte Genauigkeit der Positionsermittlung erreicht werden.

In weiterer Ausgestaltung der Erfindung weisen die Ultraschallpositionssignale eine Frequenz auf, die sich von einer Sendefrequenz des Ultraschallkopfs unterscheidet. Durch die von der Sendefrequenz des Ultraschallkopfs unterschiedliche Frequenz der Ultraschallpositionssignale wird vermieden, dass das bildgebende Ultraschallverfahren durch die Positionsermittlung beeinträchtigt wird. Eine solche Beeinträchtigung kann dann auftreten, wenn die Frequenz der Ultraschallpositionssignale und die Sendefrequenz des Ultraschallkopfs identisch oder sehr ähnlich sind. Bei einer Ausgestaltung der Erfindung werden die Ultraschallpositionssignale mit ein- und derselben Frequenz eingekoppelt/gesendet. Bei einer weiteren Ausgestaltung weisen die Ultraschallpositionssignale unterschiedliche Frequenzen auf. In weiterer Ausgestaltung der Erfindung werden die Ultraschallpositionssignale zeitlich mit dem Ultraschallsignal des Ultraschallkopfs synchronisiert eingekoppelt, beispielsweise mit einem Zeitversatz. Bei einer Ausgestaltung werden die Ultraschallpositionssignale und das Ultraschallsignal des bildgebenden Ultraschallverfahrens intermittierend eingekoppelt. Beispielsweise können anhand des empfangenen Ultraschallsignals des bildgebenden Ultraschallverfahrens sogenannte Frames erkannt und das zeitliche Senden der Ultraschallpositionssignale entsprechend angepasst und/oder synchronisiert werden, um Beeinträchtigungen des bildgebenden Ultraschallverfahrens zu vermeiden.

In weiterer Ausgestaltung der Erfindung wird der Befestigungsabschnitt mit einem komplementären Befestigungsabschnitt des Ultraschallkopfs verrastet. Alternativ oder zusätzlich wird der Befestigungsabschnitt mit einem/dem komplementären Befestigungsabschnitt zusammengesteckt. Bei dieser Ausgestaltung der Erfindung wird die Sendeeinrichtung folglich mit einer lösbaren Rastverbindung und/oder lösbaren Steckverbindung an dem Ultraschallkopf befestigt. Der Befestigungsabschnitt ist bei einer Ausgestaltung ein Rastabschnitt. Bei einer weiteren Ausgestaltung ist der Befestigungsabschnitt alternativ oder zusätzlich ein Steckabschnitt. Entsprechendes gilt mutatis mutandis für den komplementären Befestigungsabschnitt des Ultraschallkopfs. Es versteht sich, dass die Sendeeinrichtung mehrere Befestigungsabschnitte aufweisen kann. Auch der Ultraschallkopf kann mehrere komplementäre Befestigungsabschnitte aufweisen. Vorzugsweise ist der komplementäre Befestigungsabschnitt des Ultraschallkopfs durch dessen Außenkontur gebildet. Der wenigstens eine Befestigungsabschnitt der Sendeeinrichtung ist dabei auf die Außenkontur des Ultraschallkopfs abgestimmt. Hierdurch kann auf eine konstruktive Anpassung des Ultraschallkopfs zwecks Befestigung der Sendeeinrichtung verzichtet werden.

In weiterer Ausgestaltung der Erfindung weist das Verfahren einen weiteren Schritt auf: Ausgeben der Position, wobei die Position mittels einer Ausgabeeinrichtung akustisch und/oder visuell wahrnehmbar ausgegeben wird. Bei einer Ausgestaltung weist die Ausgabeeinrichtung einen Lautsprecher auf, der zur akustischen Ausgabe der Position eingerichtet ist. Bei einer weiteren Ausgestaltung weist die Ausgabeeinrichtung alternativ oder zusätzlich einen Bildschirm auf, der zur visuell wahrnehmbaren Ausgabe der Position eingerichtet ist. Beispielsweise kann die Position grafisch und/oder textbasiert ausgegeben werden. Bei einer Ausgestaltung ist die Ausgabeeinrichtung eine Komponente des bildgebenden Ultraschallsystems, wobei die Auswerteeinrichtung zur Verbindung mit der Ausgabeeinrichtung des bildgebenden Ultraschallsystems eingerichtet ist.

In weiterer Ausgestaltung der Erfindung werden ausgehend von der Kanüle keine Ultraschallsignale, im Speziellen Ultraschallpositionssignale, gesendet. Ultraschallsignale bzw. Ultraschallpositionssignale werden lediglich empfangen, nämlich mittels des wenigstens einen an der Kanüle angebrachten Sensors. Mit anderen Worten: Die Kanüle ist passiv bzw. eine passives Medizinprodukt. Im Unterschied zu einer grundsätzlich denkbaren und möglichen Einkopplung von Ultraschallsignalen ausgehend von der Kanüle ergeben sich Vorteile, insbesondere eine verbesserte Patientensicherheit.

Das erfindungsgemäße medizinische System ist zum Ermitteln einer Position einer Kanülenspitze im Inneren eines Körpers eingerichtet und weist eine Sendeeinrichtung, eine Kanüle und eine Auswerteeinrichtung auf. Vorzugsweise ist das medizinische System zum Ausführen des erfindungsgemäßen Verfahrens und/oder dessen Ausgestaltungen eingerichtet. Die Sendeeinrichtung weist mehrere Ultraschallpositionssender und wenigstens einen Befestigungsabschnitt auf. Der Befestigungsabschnitt ist zum lösbaren Befestigen an einem komplementären Befestigungsabschnitt eines Ultraschallkopfs eingerichtet. Der Ultraschallkopf ist eine Komponente eines bildgebenden Ultraschallsystems. Der Ultraschallkopf ist keine Komponente des medizinischen Systems. Die Ultraschallpositionssender sind jeweils zum Einkoppeln eines Ultraschallpositionssignals in den Körper eingerichtet. Die Kanüle weist die Kanülenspitze und wenigstens einen Sensor auf. Der Sensor ist zum Empfangen der Ultraschallpositionssignale eingerichtet. Der Sensor ist in einem bekannten axialen Abstand von der Kanülenspitze angeordnet. Vorzugsweise ist der axiale Abstand null oder jedenfalls im Hinblick auf die Positionsermittlung vernachlässigbar klein. Mit anderen Worten: Der Sensor ist vorzugsweise unmittelbar an der Kanülenspitze angeordnet. Die Auswerteeinrichtung ist mit dem Sensor und der Sendeeinrichtung verbunden. Die Verbindung kann jeweils drahtgebunden und/oder drahtlos sein. Die Auswerteeinrichtung ist zum Ermitteln von Signallaufzeiten der Ultraschallpositionssignale eingerichtet. Weiter ist die Auswerteeinrichtung zum Ermitteln der Position der Kanülenspitze in Relation zu dem Ultraschallkopf eingerichtet, wobei das Ermitteln in Abhängigkeit der Signallaufzeiten, einer definierten Relativposition der Sendeeinrichtung zu dem Ultraschallkopf und dem axialen Abstand des Sensors von der Kanülenspitze erfolgt.

Weitere Merkmale und Vorteile des medizinischen Systems ergeben sich aus der Offenbarung des erfindungsgemäßen Verfahrens und umgekehrt. Entsprechendes gilt für die jeweiligen Ausgestaltungen des erfindungsgemäßen medizinischen Systems und des erfindungsgemäßen Verfahrens.

In weiterer Ausgestaltung der Erfindung ist der wenigstens eine Befestigungsabschnitt ein Rastabschnitt, der zum Verrasten mit einer Außenkontur des Ultraschallkopfs eingerichtet ist. Alternativ oder zusätzlich ist der wenigstens eine Befestigungsabschnitt ein Steckabschnitt, der zum Aufstecken auf eine/die Außenkontur des Ultraschallkopfs eingerichtet ist. Bei dieser Ausgestaltung der Erfindung bildet die Außenkontur des Ultraschallkopfs den komplementären Befestigungsabschnitt zur Befestigung der Sendeeinrichtung. Auf eine konstruktive Anpassung des Ultraschallkopfs an das medizinische System, im Speziellen an dessen Sendeeinrichtung, kann daher verzichtet werden. Dies erlaubt einen besonders universellen Einsatz des medizinischen Systems.

In weiterer Ausgestaltung der Erfindung weist die Sendeeinrichtung wenigstens zwei Ultraschallpositionssender auf. Dies erlaubt eine ebene Positionsermittlung in Bezug auf zwei Koordinatenachsen.

In weiterer Ausgestaltung der Erfindung weist die Sendeeinrichtung wenigstens drei Ultraschallpositionssender auf. Dies erlaubt eine räumliche Positionsermittlung in Bezug auf drei Koordinatenachsen.

In weiterer Ausgestaltung der Erfindung weist die Kanüle keine Sendeeinrichtung zum Senden von Ultraschallsignalen auf, im Speziellen von Ultraschallpositionssignalen. Die Kanüle ist somit ausschließlich zum Empfangen von Ultraschallsignalen, genauer: den Ultraschallpositionssignalen, eingerichtet und weist zu diesem Zweck den wenigstens einen Sensor auf. Die Kanüle ist folglich passiv bzw. eine passives Medizinprodukt. Im Unterschied zu einer grundsätzlich denkbaren und möglichen Einkopplung von Ultraschallsignalen ausgehend von der Kanüle ergeben sich Vorteile, insbesondere eine verbesserte Patientensicherheit.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Blockdarstellung eine Ausführungsform eines erfindungsgemäßen Verfahrens zum Ermitteln einer Position einer Kanülenspitze im Inneren eines Körpers,
- Fig. 2: in schematischer Darstellung eine Ausführungsform eines erfindungsgemäßen medizinischen Systems, das zum Ausführen des Verfahrens nach Fig. 1 eingerichtet ist,
- Fig. 3: in schematisch vereinfachter Darstellung eine exemplarische Verwendungssituation des medizinischen Systems nach Fig. 2 und
- Fig. 4: in einer der Fig. 3 entsprechenden Darstellungsweise eine weitere exemplarische Verwendungssituation.

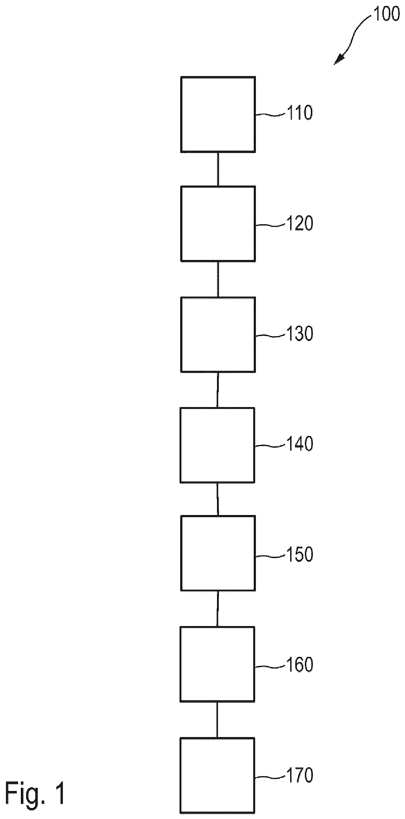

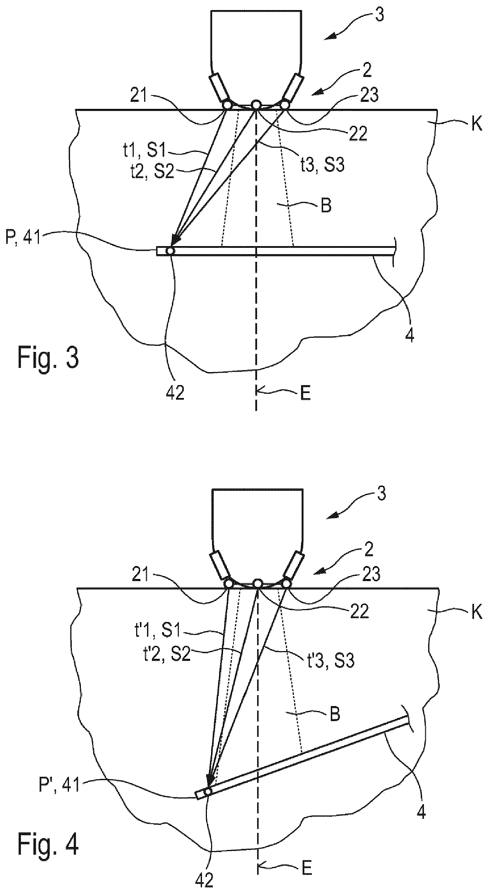

Gemäß Fig. 1 ist ein Verfahren 100 zum Ermitteln einer Position P einer Kanülenspitze 41 im Inneren eines Körpers K vorgesehen (siehe Fig. 3, 4).

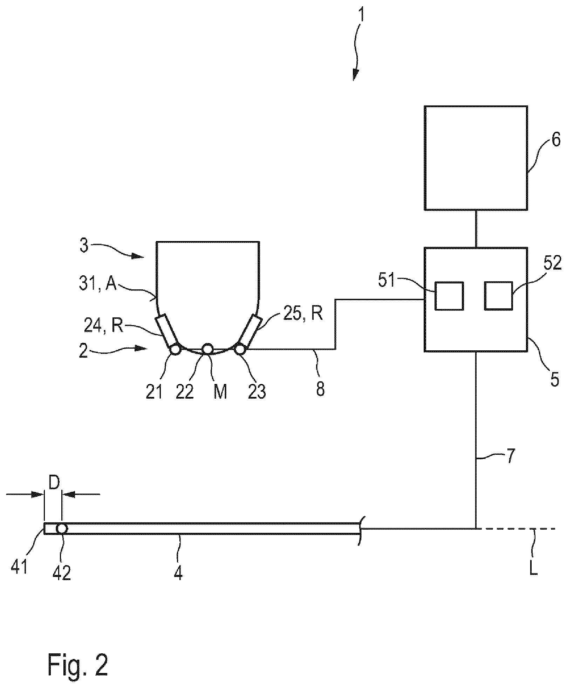

Das Verfahren 100 kann unter Verwendung des medizinischen Systems 1 nach Fig. 2 ausgeführt werden und sieht die Schritte 110 bis 170 vor.

Das medizinische System 1 weist eine Sendeeinrichtung 2, eine Kanüle 4 und eine Auswerteeinrichtung 5 auf. Bei der gezeigten Ausführungsform weist das medizinische System 1 zudem eine optionale Ausgabeeinrichtung 6 auf.

Die Sendeeinrichtung 2 weist mehrere Ultraschallpositionssender 21, 22, 23 auf. Die mehreren Ultraschallpositionssender 21, 22, 23 können auch als erster Ultraschallpositionssender 21, zweiter Ultraschallpositionssender 22 und dritter Ultraschallpositionssender 23 bezeichnet werden. Die Sendeeinrichtung 2 weist zudem wenigstens einen Befestigungsabschnitt 24, 25 auf. Die Ultraschallpositionssender 21, 22, 23 sind jeweils zum Senden eines Ultraschallpositionssignals S1, S2, S3 eingerichtet. Die Ultraschallpositionssignale S1, S2, S3 können auch als erstes Ultraschallpositionssignal S1, zweites Ultraschallpositionssignal S2 und drittes Ultraschallpositionssignal S3 bezeichnet werden. Der wenigstens eine Befestigungsabschnitt 24, 25 ist zum lösbaren Befestigen an einem komplementären Befestigungsabschnitt 31 eines Ultraschallkopfs 3 eingerichtet. Der Ultraschallkopf 3 ist nicht Bestandteil des medizinischen Systems 1, sondern stattdessen eine Komponente eines bildgebenden Ultraschallsystems, wie es im Bereich der Medizintechnik allgemein bekannt ist.

Die Kanüle 4 weist die Kanülenspitze 41 und wenigstens einen Sensor 42 auf. Die Kanülenspitze 41 ist auf übliche Weise distal angeordnet. Der Sensor 42 ist in einem bekannten axialen Abstand D von der Kanülenspitze 41 beabstandet. Der axiale Abstand D ist im Vergleich zu einer Gesamtlänge der Kanüle 4 klein. Der axiale Abstand D ist entlang einer Längsachse L der Kanüle 4 erstreckt. Bei einer in den Figuren nicht gezeigten Ausführungsform sind mehrere Sensoren an der Kanüle angebracht und in jeweils einem bekannten axialen Abstand von der Kanülenspitze positioniert.

Die Auswerteeinrichtung 5 ist mit dem Sensor 42 und der Sendeeinrichtung 2 verbunden. Zur Verbindung mit dem Sensor 42 ist eine Signalverbindung 7 vorhanden, bei der es sich vorliegend um eine drahtgebundene Verbindung handelt. Zur Verbindung mit der Sendeeinrichtung 2 ist eine weitere Signalverbindung 8 vorhanden, die ebenfalls drahtgebunden ist. Alternativ können drahtlose Verbindungen vorgesehen sein.

Das Verfahren 100 sieht in dem Schritt 110 zunächst ein Bereitstellen des Ultraschallkopfs 3 vor.

In dem Schritt 120 wird die Sendeeinrichtung 2 bereitgestellt.

In dem Schritt 130 wird die Sendeeinrichtung 2 an dem Ultraschallkopf 3 befestigt. Dabei wird die Sendeeinrichtung 2 mittels des wenigstens einen Befestigungsabschnitts 24, 25 lösbar an dem Ultraschallkopf 3 befestigt. Die Befestigung erfolgt vorliegend an dem komplementären Befestigungsabschnitt 31.

Die Sendeeinrichtung 2 wird in einer definierten Relativposition zu dem Ultraschallkopf 3 an dem Ultraschallkopf 3 befestigt. Hierdurch ist eine Position der Ultraschallpositionssender 21, 22, 23 in Relation zu dem Ultraschallkopf 3 bekannt. Die besagten Positionen der Ultraschallpositionssender 21, 22, 23 können beispielsweise in Relation zu einem Mittelpunkt M des Ultraschallkopfs 3 definiert sein, der auch als Zentrum bezeichnet werden kann.

Bei der gezeigten Ausführungsform weist die Sendeeinrichtung 2 einen ersten Befestigungsabschnitt 24 und einen zweiten Befestigungsabschnitt 25 auf. Die vorliegende Anzahl ist als rein exemplarisch zu verstehen.

Die beiden Befestigungsabschnitte 24, 25 sind bei der gezeigten Ausführungsform jeweils als Rastabschnitt R gestaltet. Die Rastabschnitte R sind unmittelbar mit einer Außenkontur A des Ultraschallkopfs 3 verrastbar. Dementsprechend bildet die Außenkontur A des Ultraschallkopfs 3 vorliegend den erwähnten komplementären Befestigungsabschnitt 31 zur lösbaren Befestigung mit den Befestigungsabschnitten 24, 25 der Sendeeinrichtung 2.

Bei einer in den Figuren nicht gezeigten Ausführungsform sind die Befestigungsabschnitte jeweils als Steckabschnitt gestaltet.

Das Verfahren 100 sieht in dem Schritt 140 ein Einkoppeln der Ultraschallsignale S1, S2, S3 in den Körper K vor.

In den gezeigten exemplarischen Verwendungssituationen (siehe Fig. 3, 4) liegt der Ultraschallkopf 3 gemeinsam mit der an ihm befestigten Sendeeinrichtung 2 auf dem Körper K auf. Für das eigentliche bildgebende Ultraschallverfahren wird mittels des Ultraschallkopfs 2 vorliegend ein Ultraschallsignal B in den Körper K eingekoppelt, das vorliegend auch als Beam bezeichnet werden kann. Die Bezeichnung "Beam" ist der Fachperson geläufig. Der Beam B wird entlang einer Bildebene E des bildgebenden Ultraschallverfahrens in den Körper K eingekoppelt. Die Bildebene E ist vorliegend entlang einer Tiefenrichtung und einer Längsrichtung erstreckt.

Im Unterschied zu dem Ultraschallsignal B des Ultraschallkopfs 3 dienen die Ultraschallpositionssignale S1, S2, S3 der Ultraschallpositionssender 21, 22, 23 nicht etwa zur Ermittlung eines Ultraschallbilds, sondern der Positionsermittlung der Kanülenspitze 41.

In dem Schritt 150 sieht das Verfahren 100 ein Empfangen der Ultraschallpositionssignale S1, S2, S3 vor. Die Ultraschallpositionssignale S1, S2, S3 werden mittels des Sensors 41 empfangen.

Das Verfahren 100 sieht in dem Schritt 160 ein Ermitteln von Signallaufzeiten t1, t2, t3 der Ultraschallpositionssignale S1, S2, S3 vor. Die Signallaufzeiten t1, t2, t3 sind in Fig. 3 grafisch mit jeweils einem Pfeil symbolisiert und können auch als erste Signallaufzeit t1, zweite Signallaufzeit t2 und dritte Signallaufzeit t3 bezeichnet werden. Die erste Signallaufzeit t1 ist die Laufzeit des ersten Ultraschallpositionssignals S1, d. h. die Zeit, die zwischen dem Senden des Ultraschallpositionssignals S1 mittels des ersten Ultraschallpositionssenders 21 und dessen Empfang mittels des Sensors 42 vergeht. Entsprechendes gilt mutatis mutandis für die zweite Signallaufzeit t2 (Laufzeit des zweiten Ultraschallpositionssignals S2) und der dritten Signallaufzeit t3 (Laufzeit des dritten Ultraschallpositionssignals S3). Die Signallaufzeiten t1, t2, t3 werden mittels der Auswerteeinrichtung 5 ermittelt. Zu diesem Zweck werden die Ultraschallpositionssignale S1, S2, S3 auf eine dem Fachmann bekannte Weise ausgewertet.

Das Verfahren 100 sieht in dem Schritt 170 ein Ermitteln der Position P der Kanülenspitze 41 in Relation zu dem Ultraschallkopf 3 vor, im Speziellen in Relation zu dem Mittelpunkt M des Ultraschallkopfs 3. Dabei wird die Position P in Abhängigkeit der Signallaufzeiten t1, t2, t3 mittels der Auswerteeinrichtung 5 ermittelt. Die Signallaufzeiten t1, t2, t3 erlauben bei bekannter Ausbreitungsgeschwindigkeit der Ultraschallpositionssignale S1, S2, S3 einen Rückschluss auf die jeweiligen Abstände zwischen den Ultraschallpositionssendern 21, 22, 23 und dem Sensor 42. Die Ermittlung der Position P erfolgt dann in Abhängigkeit der Signallaufzeiten t1, t2, t3 und auf der Grundlage einfacher geometrischer, im Speziellen trigonometrischer, Beziehungen.

In Fig. 4 ist eine weitere Verwendungssituation gezeigt, in welcher die Kanüle 4 eine in Relation zu dem Ultraschallkopf 3 unterschiedliche Lage einnimmt. Dementsprechend nimmt die Kanülenspitze 41 eine geänderte Position P' ein. Zudem ergeben sich geänderte Signallaufzeiten t'1, t'2, t'3.

Vorliegend kann die Position P (bzw. die geänderte Position P') räumlich und in Bezug auf drei Koordinatenachsen in Relation zu dem Mittelpunkt M ermittelt werden. Sind lediglich zwei Ultraschallpositionssender vorhanden, kann die Position in Bezug auf zwei Koordinatenachsen ermittelt werden, wobei dann auch von einer ebenen Positionsermittlung gesprochen werden kann.

Bei der gezeigten Ausführungsform werden die Ultraschallpositionssignale S1, S2, S3 mit einer Frequenz gesendet, die sich von einer Sendefrequenz des Ultraschallkopfs 3 und damit von der Frequenz des Ultraschallsignals B unterscheidet. Hierdurch wird vermieden, dass die Ultraschallpositionssignale S1, S2, S3 zu einer Beeinträchtigung des bildgebenden Ultraschallverfahrens führen.

Bei der gezeigten Ausführungsform sieht das Verfahren zudem ein Ausgeben der Position P mittels der Ausgabeeinrichtung 6 vor. Die Ausgabeeinrichtung 6 ist zum akustischen und/oder visuell wahrnehmbaren Ausgeben der Position P eingerichtet. Zu diesem Zweck kann die Ausgabeeinrichtung 6 beispielsweise einen Lautsprecher (akustische Ausgabe) oder einen Bildschirm aufweisen (visuelle Ausgabe). Die Ausgabeeinrichtung 6 ist bei einer Ausgestaltung des bildgebenden Ultraschallsystem zugeordnet, das auch den Ultraschallkopf 3 umfasst. In diesem Fall ist die Auswerteeinrichtung 5 mit der Ausgabeeinrichtung 6 verbindbar.

Vorliegend weist die Auswerteeinrichtung 5 zudem eine Speichereinheit 51 und eine Prozessoreinheit 52 auf. Die Prozessoreinheit 52 ist wenigstens zum Ausführen der Schritte 160, 170 eingerichtet.

## Patentansprüche

1. Verfahren (100) zum Ermitteln einer Position (P) einer Kanülenspitze (41) im Inneren eines Körpers (K), aufweisend die Schritte:
Bereitstellen (110) eines Ultraschallkopfs (3) eines bildgebenden Ultraschallsystems;
Bereitstellen (120) einer Sendeeinrichtung (2), die mehrere Ultraschallpositionssender (21, 22, 23) und wenigstens einen Befestigungsabschnitt (24, 25) aufweist;
Befestigen (130) der Sendeeinrichtung (2) an dem Ultraschallkopf (3), wobei die Sendeeinrichtung (2) mittels des Befestigungsabschnitts (24, 25) lösbar und in einer definierten Relativposition zu dem Ultraschallkopf (3) an dem Ultraschallkopf (3) befestigt wird;
Einkoppeln (140) mehrerer Ultraschallpositionssignale (S1, S2, S3) in den Körper (K), wobei die Ultraschallpositionssignale (S1, S2, S3) mittels der mehreren Ultraschallpositionssender (21, 22, 23) eingekoppelt werden;
Empfangen (150) der Ultraschallpositionssignale (S1, S2, S3), wobei die Ultraschallpositionssignale (S1, S2, S3) mittels wenigstens eines Sensors (42) empfangen werden, der an einer in dem Körper (K) befindlichen Kanüle (4) angebracht und in einem bekannten axialen Abstand (D) zu deren Kanülenspitze (41) angeordnet ist;
Ermitteln (160) von Signallaufzeiten (t1, t2, t3) der Ultraschallpositionssignale (S1, S2, S3), wobei die Signallaufzeiten (t1, t2, t3) mittels einer Auswerteeinrichtung (5) ermittelt werden, die mit dem Sensor (42) und der Sendeeinrichtung (2) verbunden ist;
Ermitteln (170) der Position (P) der Kanülenspitze (41) in Relation zu dem Ultraschallkopf (3), wobei die Position (P) in Abhängigkeit der Signallaufzeiten (t1, t2, t3), der Relativposition der Sendeeinrichtung (2) zu dem Ultraschallkopf (3) und dem axialen Abstand (D) des Sensors (42) von der Kanülenspitze (41) mittels der Auswerteeinrichtung (5) ermittelt wird.

2. Verfahren (100) nach Anspruch 1, wobei wenigstens ein erstes Ultraschallpositionssignal (S1) mit einem ersten Ultraschallpositionssender (21) eingekoppelt wird und ein zweites Ultraschallpositionssignal (S2) mit einem zweiten Ultraschallpositionssender (22) eingekoppelt wird.

3. Verfahren (100) nach Anspruch 2, wobei ein drittes Ultraschallpositionssignal (S3) mit einem dritten Ultraschallpositionssender (23) eingekoppelt wird.

4. Verfahren (100) nach einem der vorhergehenden Ansprüche, wobei die Ultraschallpositionssignale (S1, S2, S3) eine Frequenz aufweisen, die sich von einer Sendefrequenz des Ultraschallkopfs (3) unterscheidet.

5. Verfahren (100) nach einem der vorhergehenden Ansprüche, wobei der Befestigungsabschnitt (24, 25) mit einem komplementären Befestigungsabschnitt (31) des Ultraschallkopfs (3) verrastet und/oder zusammengesteckt wird.

6. Verfahren (100) nach einem der vorhergehenden Ansprüche, weiter aufweisend den Schritt:
Ausgeben der Position (P), wobei die Position (P) mittels einer Ausgabeeinrichtung (6) akustisch und/oder visuell wahrnehmbar ausgegeben wird.

7. Verfahren (100) nach einem der vorhergehenden Ansprüche, wobei ausgehend von der Kanüle (4) keine Ultraschallsignale gesendet werden.

8. Medizinisches System (1) zum Ermitteln einer Position (P) einer Kanülenspitze (41) im Inneren eines Körpers (K), aufweisend
eine Sendeeinrichtung (2) mit mehreren Ultraschallpositionssendern (21, 22, 23) und wenigstens einem Befestigungsabschnitt (24, 25), wobei der Befestigungsabschnitt (24, 25) zum lösbaren Befestigen an einem komplementären Befestigungsabschnitt (31) eines Ultraschallkopfs (3) eines bildgebenden Ultraschallsystems eingerichtet ist, und wobei die Ultraschallpositionssender (21, 22, 23) zum Einkoppeln jeweils eines Ultraschallpositionssignals (S1, S2, S3) in den Körper (K) eingerichtet sind,
eine Kanüle (4) mit der Kanülenspitze (41) und wenigstens einem Sensor (42), der zum Empfangen der Ultraschallpositionssignale (S1, S2, S3) eingerichtet ist und in einem bekannten axialen Abstand (D) von der Kanülenspitze (41) angeordnet ist, und
eine Auswerteeinrichtung (5), die mit dem Sensor (42) und der Sendeeinrichtung (2) verbunden ist, wobei die Auswerteeinrichtung (5) eingerichtet ist zum Ermitteln von Signallaufzeiten (t1, t2, t3) der Ultraschallpositionssignale (S1, S2, S3) und zum Ermitteln der Position (P) der Kanülenspitze (41) in Relation zu dem Ultraschallkopf (3), wobei das Ermitteln in Abhängigkeit der Signallaufzeiten (t1, t2, t3), einer definierten Relativposition der Sendeeinrichtung (2) zu dem Ultraschallkopf (3) und dem axialen Abstand (D) des Sensors (42) von der Kanülenspitze (41) erfolgt.

9. Medizinisches System (1) nach Anspruch 8, wobei der wenigstens eine Befestigungsabschnitt (24, 25) ein Rastabschnitt (R) ist, der zum Verrasten mit einer Außenkontur (A) des Ultraschallkopfs (3) eingerichtet ist, oder ein Steckabschnitt ist, der zum Aufstecken auf eine Außenkontur (A) des Ultraschallkopfs (3) eingerichtet ist.

10. Medizinisches System (1) nach Anspruch 8 oder 9, wobei die Sendeeinrichtung (2) wenigstens zwei Ultraschallpositionssender (21, 22) aufweist.

11. Medizinisches System (1) nach einem der Ansprüche 8 bis 10, wobei die Sendeeinrichtung (2) wenigstens drei Ultraschallpositionssender (21, 22, 23) aufweist.

12. Medizinisches System (1) nach einem der Ansprüche 8 bis 11, wobei die Kanüle (4) keine Sendeeinrichtungen zum Senden von Ultraschallsignalen aufweist.
